# EUROPEAN PATENT SPECIFICATION

(11) **EP 0 496 385 B1**
(45) Date of publication and mention of the grant of the patent: **05.04.1995**
(21) Application number: 92101028.6
(22) Date of filing: 22.01.1992
(51) Int. Cl.: C07C 249/04

(54) **Multistep process for the liquid phase ammoximation of carbonyl compounds**
Mehrstufiges Verfahren zur Ammoximation in der flüssigen Phase von carbonylischen Verbindungen
Procédé à étapes multiples d'ammoxymation en phase liquide des composés carbonyliques

(30) Priority: 23.01.1991 IT MI910144
(43) Date of publication of application: 29.07.1992
(73) Proprietor: ENICHEM S.p.A., 20124 Milano (IT)
(72) Inventor: Tonti, Sergio, Dr., I-30173 Mestre, Venice (IT); Roffia, Paolo, Dr., I-21047 Saronno, Varese (IT); Gervasutti, Vittorio, I-30174 Mestre, Venice (IT)
(74) Representative: Weinhold, Peter, Dr.

(56) References cited:
- EP-A- 0 267 362
- EP-A- 0 299 430
- EP-A- 0 347 926
- EP-A- 0 384 390

## Description

The present invention relates to a multistep process for the liquid phase ammoximation of carbonyl compounds with hydrogen peroxide and ammonia. A typical example is the ammoximation of cyclohexanone to cyclohexanone-oxime, and in the following reference will be almost always made to this particular type of process; of course, this does not exclude the applicability of the process according to the invention to other carbonyl compounds.

EP-A-208,311; 267,362; 299,430 and 347,926 teach that said ammoximation can effectively be carried out in the presence of a catalyst based on silicon and titanium. This type of catalyst permits to obtain very high conversions and selectivities; nevertheless, a quantitative conversion (which would simplify the oxime separation and recovery) is practically never obtained, particularly in the case of cyclohexanone, and the unreacted carbonyl compound poses a problem not only because it is necessary to recover it, but also owing to the possible secondary reaction which it can give rise to (during the separation and purification of the oxime). The by-products of these reactions (in the case of cyclohexanone these are cyclohexyl-cyclohexanone, bis-cyclohexenyl-cyclohexanone and octahydro-phenazine), as is known, adversely affect the quality of the caprolactam obtainable in the subsequent Beckmann rearrangement. In order to complete the oximation of the residual ketone, it is possible to resort to a reaction with a solution of hydroxylamine sulphate, under operative conditions known from the art. However, on the one hand, the problem of the non-quantitative conversion of the carbonyl compound is present in both the ammoximation of cyclohexanone to cyclohexanone-oxime and the ammoximation of other ketones and aldehydes such as e.g. acetone, methylethyl ketone (2-butanone), acetophenone, cyclododecanone, enantic aldehyde (1-heptanal), etc. and, on the other hand, the hydroxylamine sulphate solutions are obtainable only by complex processes such as, for example, the Raschig process (reduction of the nitrogen oxides with ammonium bisulphite).

There has now been found an ammoximation process which permits to reduce the amount of residual ketone (or of residual aldehyde) contained in the effluents of the initial step(s) of the ammoximation process to those levels which can be reached with hydroxylamine sulphate, without resorting, however, to the use of said sulphate which, as already pointed out, can be prepared only be means of quite complex processes.

Accordingly, in its broadest aspect the present invention relates to a multistep process for the liquid phase ammoximation of carbonyl compounds with H₂O₂ and NH₃, at 60 - 100°C and 1.5 - 5 bar and in the presence of a (suspended) catalyst based on silicon, titanium and oxygen, wherein:
a) in one or more initial steps the molar ratio H₂O₂ : carbonyl compound ranges from 0.9:1 to 1.15:1 (preferably from 1.0:1 to 1.1:1) and the carbonyl compound conversion is effected up to at least 95% (preferably up to 96 - 99%);
b) in a final (last) step (exhaustion step) the molar ratio H₂O₂ : carbonyl compound is at a higher level, i.e., ranges from 1.5:1 to 3.0:1 (preferably from 1.5:1 to 2.2:1).

The above ranges are critical for the purpose of improving the process. It has also been tried to effect the quantitative conversion of the carbonyl compound in a single reactor, without any additional completion (exhaustion) step and by using much higher amounts of oxidant (H₂O₂) already at the beginning, as well as much longer reaction times, but it was found that the initial substantial excess of H₂O₂ and the too long reaction times cause an instability of the reaction system. Namely, competitive reactions were observed, which involved the oxime and/or the carbonyl compound and/or the ammonia. The competitiveness, obviously, refers to the ammoximation reaction. Said competitive (secondary) reactions led to a reduction of the quality of the oxime produced and to a considerable formation of nitrogen-containing by-products (N₂O, N₂, NO₂⁻, NO₃⁻ etc.). A consequence of this quality reduction was that the quality specifications of the caprolactam obtainable from the oxime (in cascade) were not met. In other words, it has surprisingly been found that it is possible to employ a large excess of H₂O₂, provided said excess is added only after a certain conversion level has been reached, i.e. provided that the residual carbonyl compound concentrations are very low or, in other words, the carbonyl compound conversion has reached at least 95%. In fact it was observed that under these conditions the expected quality reduction did not occur at all and that a practically complete conversion of the carbonyl compound was obtained without having to carry out the undesired and complex post-treatments (with hydroxylamine sulphate) and without secondary reactions taking place. The practically quantitative reaction of the carbonyl compound provided by the process of the invention results in the production of an oxime of equivalent of higher quality than that obtainable according to the prior art. A few preferred operative details are briefly listed hereinafter.
A) Residual Reagent in the Effluent from the Initial Step(s)
   Particularly good results, in terms of yield calculated on hydrogen peroxide and of quality of the oxime produced, may be obtained when the concentration of the residual carbonyl compound in the effluent from the initial step(s) does not exceed 1 and, preferably, 0.5% by weight.
B) Operative Conditions of the Initial Step(s)
   The ammonia concentration in the liquid reaction medium preferably ranges from 1.0 to 2.5% (particularly from 1.5 to 2.0%) by weight. The molar ratio H₂O₂ : ketone (or aldehyde) preferably ranges from 1.0:1 to 1.1:1. The concentration of the catalyst suspended in the liquid medium usually is such as to result in a specific productivity (expressed as parts by weight of oxime produced per part of catalyst and per hour) of from 6 to 12, preferably of about 8. The residence time in each of the initial steps generally does not exceed 120 minutes and preferably ranges from 30 to 90 minutes.
C) Exhaustion Step
   In the last step (exhaustion step), where an (almost) complete conversion of the residual carbonyl compound is to be effected, in particular with a concentration of the unreacted compound not higher than 200 ppm (preferably not higher than 100 ppm and, still more preferably, not higher than 50 ppm on the liquid medium), the above parameters, as already mentioned, are in the following ranges:
   - molar ratio H₂O₂ : carbonyl compound from 1.5:1 to 3:1 (preferably from 1.5:1 to 2.2:1);
   - residence time preferably from 10 to 60 minutes.

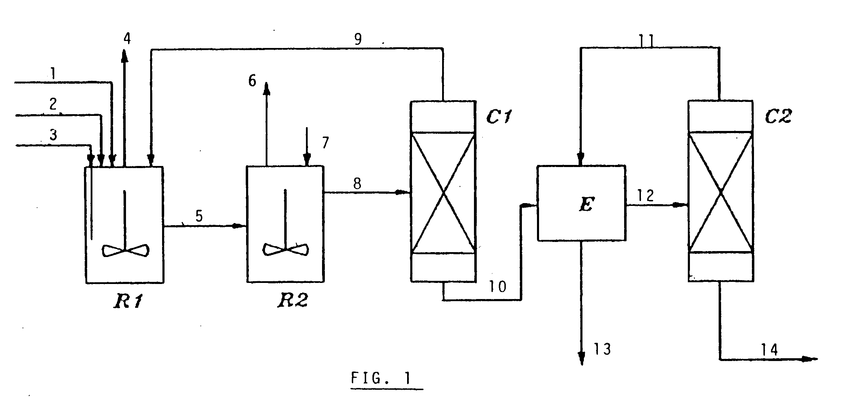

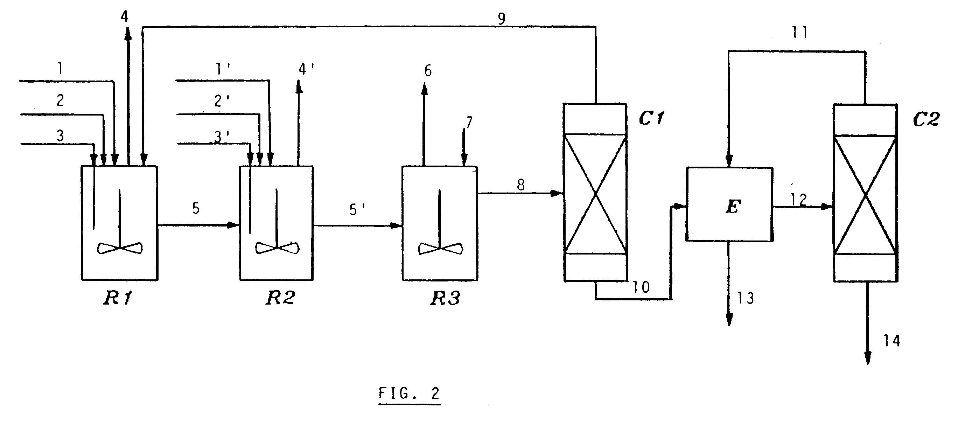

   It is preferred to not introduce further fresh ammonia in the exhaustion step since the amount dissolved in the liquid is sufficient for the above purpose; too large an ammonia excess, referred to the carbonyl compound, in the presence of a hydrogen peroxide excess could result in a loss of oxidant and in the formation of undesired gaseous by-products, such as N₂ and N₂O. In the exhaustion step, the specific catalyst productivity usually decreases to a lower level, i.e., from 0.1 to 5 (preferably from 0.3 to 0.5), due to the different operative conditions, and the temperature is preferably maintained at the value specified above for the initial step(s).
D) General Considerations
   As already mentioned, the temperature in all of the steps ranges from 60 to 100°C (preferably from 70 to 90°C). At lower temperatures the reaction is rather slow while at higher temperatures the negative effect of the parallel reactions as well as of the consecutive reactions (which start from already formed oxime) begins to become noticeable. The pressure in each of the initial step(s) and in the exhaustion step is to prevent the reaction liquid to start boiling and is to maintain the ammonia concentration in the liquid medium at from 1 to 2.5% by weight, preferably at a value lower than 2%. The pressure also acts as motive power in the liquid filtration. Pressures of from 1.5 to 5 bar (preferably from 1.8 to 3 bar) are sufficient, with the pressures decreasing from the first to the last step. The residence time in each step, with exception of the last step, is to be such as to result in a residual ketone or residual aldehyde conversion of equal to or higher than 95%. The reaction time, in each of these steps, is generally not longer than one hour in order to prevent subsequent reactions of the oxime which has formed. Conversely, too short reaction times lead to an unsatisfactory conversion of the carbonyl compound and to too high a concentration of the reagent in the liquid medium, which promotes the formation of by-products through condensation reactions. In the last step (exhaustion step), the reaction time usually is much shorter in consideration of the lower amount of ketone (aldehyde) to be converted. The molar ratio hydrogen peroxide/ketone (aldehyde) in each step, with exception of the exhaustion step, preferably is slightly above unity, since a small amount of hydrogen peroxide is always consumed, as already mentioned, in parallel reactions (with formation of gaseous products such as N₂ and N₂O, by ammonia oxidation). Furthermore, as already pointed out, the molar ratio hydrogen peroxide/carbonyl compound in the last step, where it is no longer advisable to feed carbonyl compound and which is to bring the carbonyl compound concentration preferably to values below 200 and particularly below 100 ppm, is considerably higher than that utilized in the preceding step(s) (from 1.5:1 to 3:1 and preferably from 1.5:1 to 2.2:1). The productivity of each step is strictly related to the concentration of the catalyst suspended in the solution contained in each reactor. The continuous feeding to each step is preferably controlled in order to have a specific productivity (expressed in parts of produced oxime per part of catalyst and per hour) within the prefixed values. In order to ensure an effective dispersion of the catalyst in the liquid medium, the catalyst concentration usually ranges from 1 to 15% by weight. At too low a concentration the productivity of each step becomes too low and no profitable in economic respect while too high a concentration gives rise to problems as regards stirring and/or filtration of the reaction product. Preferably and advantageously said concentration is maintained at 1 to 6% by weight. As catalyst it is possible to use, e.g., a titanium silicalite as described in EP-A-267,362 and 299,430, or one of the amorphous compounds described in EP-A-347,926. The average particle size of the catalyst generally ranges from 1 to 100 »m preferably from 5 to 50 »m.
E) Solvents
   Suitable solvents for the ammoximation (including the exhaustion step) are the usual organic solvents described in the documents mentioned above, a few of which have been cited hereinbefore. Said solvents can be both water-soluble and water-insoluble, provided they are stable (under the reaction conditions) towards hydrogen peroxide and exhibit a good dissolving power towards the oximes, in particular towards cyclohexanone-oxime. In the case of many oximes it is possible to operate also in an aqueous medium, but cyclohexanone-oxime, owing to its low water-solubility, would tend to deposit on the catalyst, thereby inhibiting the catalyst activity once the saturation limit has been reached. Therefore it is advantageous to use organic solvents in order to obtain a high specific productivity of the catalyst and of the reactor. Suitable solvents are, for example, tertiary alcohols, which are stable to hydrogen peroxide, in particular t-butyl alcohol, miscible in any ratio with water, cyclo-hexanol, and aromatic compounds such as benzene, toluene, xylenes, chlorobenzene, mixtures thereof, etc. If water-immiscible solvents are utilized, the presence of three phases is observed: an aqueous phase (water is produced by the reaction), an organic phase (which maintains in solution most of the oxime produced) and a solid phase which is suspended between the two liquid phases and comprises the catalytic system. In all of the following examples t-butyl alcohol is employed as solvent; however, that does not exclude the possibility of using other solvents which are stable towards hydrogen peroxide (either water-soluble or water-insoluble). Particularly advantageous results may be obtained, for example, by substituting toluene for t-butanol. Due to the low water solubility of cyclohexanone-oxime, it is advisable to limit the amount of water present to that which forms (during the reaction) and to that which probably must be recycled with the solvent. t-Butyl alcohol, for example, which is separated and usually recycled on conclusion of the reaction, has the composition of the aqueous azeotrope (about 12% by weight of water). The oxime concentration gradually rises in each step and its maximum value, when an organic solvent is employed, usually ranges from 10 to 30%, preferably from 20 to 25% by weight. Although it is economically profitable to operate with a maximum oxime concentration this is not too advisable since when said concentration exceeds certain values there is an interference with the subsequent oxime reactions, leading to the formation of by-products which strongly affect the quality thereof. The weight ratio of solvent to carbonyl compound generally ranges from 2.5:1 to 10:1.
F) Operative Details
   The process according to the invention and the recovery of the oxime from the solution leaving the last step (exhaustion step), in which the residual carbonyl compound concentration is reduced to a value lower than 200 ppm and even lower than 100 ppm, can be carried out according to the schemes shown in figure 1 and figure 2, which are given for merely illustrative purposes, without limiting, however, the scope of the invention.
   According to figure 1, a carbonyl compound, e.g. cyclohexanone (1), hydrogen peroxide (2), ammonia (3) and a t-butanol make-up (now shown in the figure) enter a primary reactor R1, equipped with a stirrer, a filtering element (not shown in the figure) and a venting device (4), where the ammoximation reaction is effected up to very high values of conversion (up to above 95% of conversion). The reaction mixture (5) is then conveyed into a second step reactor R2 (exhaustion reactor), which also is equipped with a vent (6) and is charged with an excess of hydrogen peroxide (7). The final effluent (8) (practically free of residual carbonyl compound and containing t-butanol, oxime and ammonia) is passed to a distillation column C1. From the column head, the ammonia and all of the solvent (t-butanol in the form of an azeotrope containing 12% by weight of water) are recovered; the ammonia-azeotrope mixture (9) is recycled to the 1st step (primary step). From the bottom of the column C1, a liquid (10) composed of water and oxime is recovered and is then subjected to extraction in an apparatus E fed with toluene (11). All of the oxime migrates to the toluene phase, and from a subsequent separator, not shown in the figure, the toluene phase (12) is withdrawn and sent to a column C2 for solvent distillation and oxime dehydration. From said separator (not shown), a water phase (13), containing most of the water-soluble foreign matters, is discharged. From the head of column C2 toluene is recovered in the form of an azeotropic mixture with the reaction water; after a demixing (not shown in the figure), toluene (11) is recycled to the extraction section. The anhydrous oxime (14) which leaves the bottom of column C2 is passed to the Beckmann rearrangement for the production of, e.g., caprolactam.

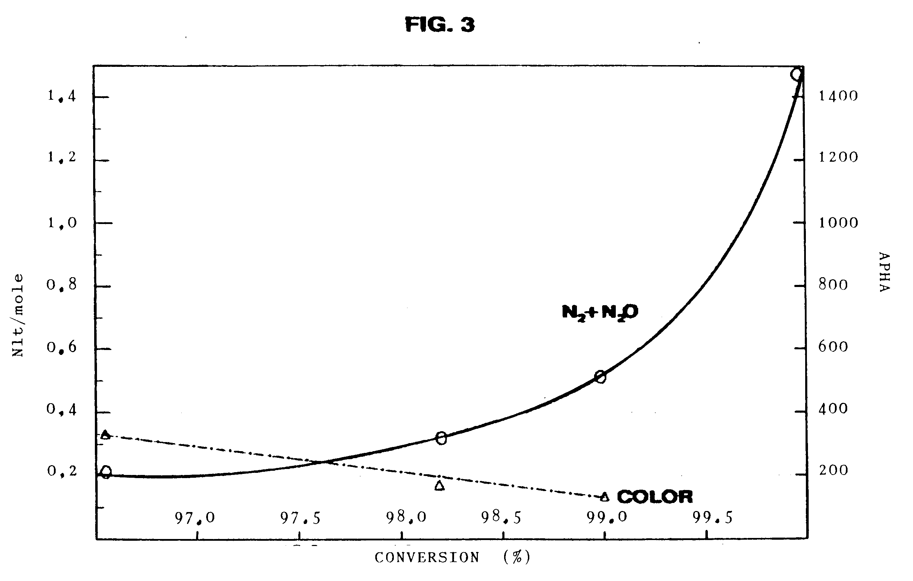

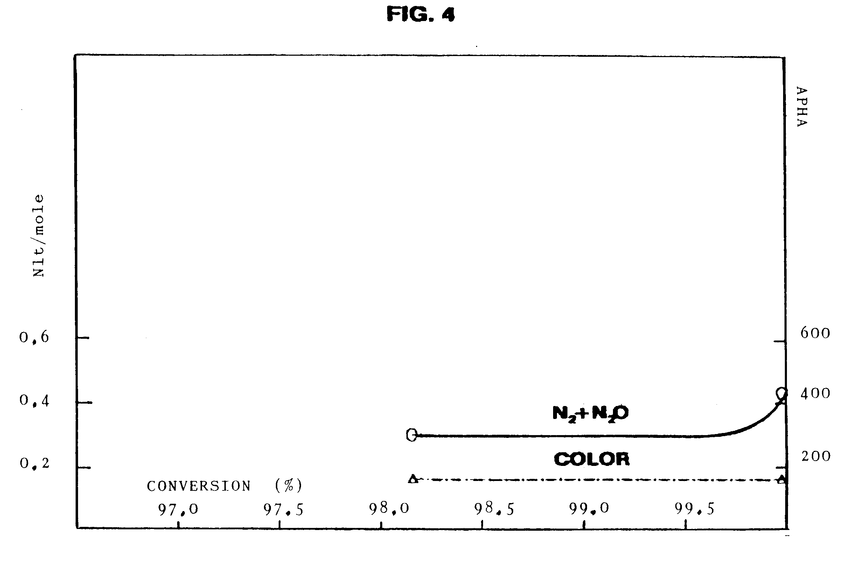

   Figure 2 illustrates, by means of analogous symbols, the case in which, instead of one primary step, there are two primary steps; figure 3 and figure 4 concern the results of some tests and will be discussed in the examples.
G) Apparatus
   The invention can advantageously be carried out in reactors arranged in series and stirred in order to maintain in suspension the catalyst which is insoluble in the liquid medium. The most suitable reactor is that known as CSTR (Continuous Stirred Tank Reactor). This type of reactor ensures an effective dispersion of the catalyst system and at the same time, on the basis of a suitable regulation of the residence times, the desired conversion of the carbonyl compound, the residual concentration of which is to not exceed certain optimum values, beyond which the already cited formation of undesired by-products takes place (which adversely affect the oxime quality and render the oxime not or less acceptable for the conversion to lactam). Ammonia, hydrogen peroxide and carbonyl compound (in particular cyclohexanone) are continuously fed to each reactor of the initial steps and the temperature is maintained around the desired value (by means of cooling, since the reaction is exothermic). The reaction heat can be removed indirectly (through a heat exchanger arranged inside the reactor) or by causing the reaction liquid to circulate in a refrigerated circuit outside the reactor (loop reactor). Each reactor should be equipped with a vent for removing small amounts of gases (N₂, O₂, N₂O) which form as reaction by-products by direct ammonia oxidation. It is advisable to mount on said vent a scrubber for the small amounts of solvent which may be entrained by the gaseous compounds. It also is necessary to install in each reactor a filtering system and a purge for the exhausted catalyst. The filtering system, which is arranged inside the reactor or in a circuit outside the reactor, permits to separate the liquid phase from the catalyst, which remains in the reactor, while the filtered liquid is sent to another reactor or to a distillation column (in the case of the last step) for the oxime recovery. It is preferable to install - coupled to the filtering system - a device for the discontinuous purging of exhausted catalyst, which is to be replaced by a fresh catalyst make-up in order to maintain the desired catalytic activity in each step. The cyclohexanone feeding, however, is not provided for in the last step, since the specific purpose of this step is to complete the conversion of the carbonyl compound. Therefore, the reaction liquid directly flows to the oxime recovery section, without undergoing further treatments. The particle size of the (crystalline or amorphous) catalyst, of the order of tens of »m, permits, on the one hand, an easy dispersion thereof in the reaction medium and, on the other hand, an easy separation, by means of usual filtering systems, from the reaction medium. The following examples are given for merely illustrative purposes and are not to be construed as a limitation of the scope of the invention.

### EXAMPLE 1 (COMPARATIVE) - Initial Ammoximation

To a 1 liter reactor, equipped with stirrer and continuous feeding and discharge systems, there were continuously fed:
- cyclohexanone = 70.6 g/h;
- t-butyl alcohol (TBA) (containing about 12% by weight of H₂O) = 232.5 g/h;
- hydrogen peroxide (at 49.7% by weight) = 54.2 g/h (H₂O₂ : ketone feeding molar ratio = 1.10:1);
- gaseous ammonia = amount sufficient to maintain a constant concentration (about 2% by weight calculated on the liquid medium).

The level of the liquid was maintained constant by adjusting an average residence time of 72 minutes (± 1 min), and the catalyst concentration was maintained constant around 2% by weight (calculated on the liquid medium). The catalyst was composed of spheroidal titanium silicalite (suspended in the liquid) having an average particle size of about 20 »m. The reaction temperature was maintained constant at 85°C (± 1°C) by means of a thermostatic fluid circulating in the reactor jacket; the operating pressure was 2.3 bar. The resulting product was continuously withdrawn through a stainless steel element equipped with a porous baffle and arranged inside the reaction (dimension of the pores = 5 »m), in order to prevent the passage of the catalyst; under regular operating conditions the product leaving the reactor had the following composition:

| | |
|---|---|
| - cyclohexanone-oxime | 21.0% by weight |
| - cyclohexanone | 0.3% by weight |
| - water | 22.0% by weight |
| - ammonia | 2.0% by weight |
| - solvent (TBA) | the balance to 100%. |

This corresponded to the following results:

| | |
|---|---|
| - cyclohexanone conversion | 98.3% |
| - cyclohexanone selectivity to oxime | 99.6% |
| - H₂O₂ conversion | 100.0% |
| - H₂O₂ selectivity to oxime | 89.1%. |

Data and results are reported in figure 3 and in Table 1 below, where also the gaseous by-products (N₂ + N₂O) and the color (APHA) are indicated. Said APHA color can be determined, as is known, according to ASTM-D-1209/69.

### EXAMPLE 1/BIS (COMPARATIVE)

Example 1 was repeated, increasing the (H₂O₂ : ketone) feed molar ratio up to a value of 1.15:1. The results, which are reported in Table 1 below and graphically represented in figure 3, show that an increase in the hydrogen peroxide amount results in a small reduction of the product color, while it causes a not acceptable increase of the (gaseous) by-products deriving from ammonia oxidation, in particular of N₂ and N₂O. Conversely, if said ratio is reduced to values below 1.10:1, lower amounts of gaseous by-products, but also much higher (APHA) color values are obtained, as is shown in figure 3, where the amount of by-products is expressed as N l./mole (normal litres per mole of oxime present in the reaction system).

### EXAMPLE 2 (COMPARATIVE) - Completion of Ammoximation with Hydroxylamine Sulphate; Integrated Ammoximation

Example 1 was repeated and the effluent from the reactor was directly fed to an azeotropic distillation column, from the top of which the solvent (t-butanol containing about 12% by weight of H₂O) was recovered; from the bottom there was recovered a mixture having the following composition:

| | |
|---|---|
| - cyclohexanone-oxime | 58.6 % by weight |
| - cyclohexanone | 0.85% by weight |
| - water | 40.5 % by weight |

Said tail mixture was continuously fed to a second (stirred) CSTR reactor, to which there was also fed an aqueous solution of hydroxylamine sulphate of formula (NH₃OH)₂SO₄, hereinafter referred to as HYXAS, at a concentration of 10% by weight. The hydroxylamine amount was such as to maintain a NH₂OH/cyclohexanone molar ratio of 2/1. The pH was maintained constant at about 4 (± 0.1) by adding an aqueous ammmonia solution (at 15% by weight). The temperature was maintained at 90°C (±1°C).

The average residence time was 15 minutes (± 1 min), thereby obtaining a cyclohexanone-oxime having a maximum concentration of residual cyclohexanone lower than 100 ppm. The effluent from the reactor was sent to a phase separator where (after a residence time sufficient to obtain a sharp phase separation) a molten cyclohexanone-oxime phase, containing 6.5% by weight of water, and an aqueous saline phase were obtained. Said oxime was then dehydrated and subjected to the Beckmann rearrangement; data and results are reported in Table 1.

### EXAMPLE 3

To a first step (initial ammoximation step) there were fed, under the operative conditions of example 1:

| | |
|---|---|
| - cyclohexanone | 70.6 g/h |
| - TBA (12% of H₂O) | 232.5 g/h |
| - hydrogen peroxide (49.7%) (H₂O₂ : ketone feed molar ratio = 1.10:1) | 54.2 g/h |
| - ammonia: amount sufficient to maintain a constant concentration (about 2% by weight) in the liquid medium. | |

The effluent from this first step, equal to 300 g/h, having the following composition:

| | |
|---|---|
| - cyclohexanone-oxime | 21.0 % by weight |
| - cyclohexanone | 0.30% by weight |
| - water | 22.0 % by weight |
| - ammonia | 2.0 % by weight |

was fed to a second reactor (exhaustion reactor) similar to the first one and maintained at a constant operation pressure of 1.8 bar and at a temperature of 85°C. To said reactor an aqueous solution of hydrogen peroxide at 50% by weight was also fed. The amount of solution was 1.6 g/hour, corresponding to a H₂O₂/residual ketone molar ratio (exhaustion ratio) equal to 2.02:1. Also in this second step (exhaustion step) it was operated with a catalyst in suspension (titanium silicalite) in an amount equal to about 2% by weight of the solution contained in the reactor. The average residence time was 30 minutes (± 1 min). The reaction product leaving the second reactor tor (through the filtering element) exhibited the following composition:

| | |
|---|---|
| - cyclohexanone-oxime | 21.3% by weight |
| - cyclohexanone | less than 100 ppm |
| - water | 23.2% by weight |
| - ammonia | 1.7% by weight |
| - solvent | the balance to 100%. |

Considering the total amounts of reagents fed, the H₂O₂/cyclohexanone total molar ratio was 1.13:1. The cyclohexanone conversion was equal to 99.95% and the selectivity of cyclohexanone to cyclohexanone-oxime was higher than 99%. The hydrogen peroxide conversion was practically quantitative and the selectivity of hydrogen peroxide to oxime was 87.4%. After separation of the solvent by distillation and after dehydration of the resulting oxime, there was obtained, by Beckmann rearrangement, a caprolactam satisfying (after purification) the quality specifications required by the market (optical density, at 290 nm, lower than 0.05; permanganate number higher than 20,000 seconds; volatile bases below 0.5 milli-equivalents/kg). It is evident that by operating according to the invention it is possible to obtain excellent results without having to utilize a reagent alien to the ammoximation reaction (for example hydroxylamine sulphate). The very small amount of gaseous by-products (0.41 N l./mole) and the final color (about 180 APHA) are graphically represented in figure 4, which permits to immediately recognize the technical importance of the invention. The intersection of the two curves in figure 3 practically indicates an optimum conversion level, beyond which it is advisable to pass to the exhaustion step (with very high H₂O₂ : ketone ratios). It was impossible to foresee the range corresponding to the best results (95-99%).

### EXAMPLE 4 - Three-Step Process

The reactor of example 1 was fed with

| | |
|---|---|
| - cyclohexanone | 35.3 g/h |
| - TBA (12% H₂O) | 232.5 g/h |
| - hydrogen peroxide (50%) (H₂O₂ : ketone feed ratio = 1.04:1) | 25.5 g/h |
| - gaseous ammonia: amount sufficient to maintain constant its concentration (about 2% by weight calculated on the liquid medium). | |

The liquid level in the reactor was maintained constant and the average residence time was 60 minutes (± 1 min). The catalyst (titanium silicalite) concentration in the reactor was maintained constant (about 2% by weight calculated on the reaction medium). Also the reaction temperature was maintained constant at 85°C (± 1°C), by means of a thermostatic fluid circulating in the reactor jacket; the pressure was equal to 2.8 bar. The cyclohexanone conversion was 97.8%. The composition of the effluent from the first step was as follows:

| | |
|---|---|
| - cyclohexanone-oxime | 13.0 % by weight |
| - cyclohexanone | 0.26% by weight |
| - ammonia | 2.0 % by weight |
| - water | 18.2 % by weight. |

The product from this first step was passed to a second reactor, identical with the preceding one, and simultaneously there were fed:

| | |
|---|---|
| - cyclohexanone | 35.3 g/h |
| - hydrogen peroxide (at 50% by weight) (H₂O₂ : ketone feed ratio = 1.06:1) | 26.0 g/h |
| - gaseous ammonia: amount sufficient to maintain constant its concentration (about 2% by weight). | |

The operative conditions of the second step were:

| | |
|---|---|
| - temperature | 85°C (± 1°C) |
| - pressure | 2.3 bar |
| - catalyst in suspension | 2% by weight |
| - average residence time | 60 minutes (± 1 min). |

The effluent from the second step, equal to 373 g/h, had the following composition:

| | |
|---|---|
| - cyclohexanone-oxime | 21.4 % by weight |
| - cyclohexanone | 0.30% by weight |
| - ammonia | 2.05% by weight |
| - water | 22.1 % by weight. |

Said effluent (from the second step) was fed to a third reactor (exhaustion reactor), similar to the reactors of the first and second steps, operating under the following conditions:

| | |
|---|---|
| - temperature | 85°C (± 1°C) |
| - pressure | 1.8 bar |
| - concentration of the catalyst in suspension | about 2% by weight |
| - average residence time | 30 minutes. |

Said third reactor was fed with 8 g/h of hydrogen peroxide (at 10% by weight), corresponding to an exhaustion ratio of 2.06:1.

The product of the third step, equal to 380 g/h, had the following composition:

| | |
|---|---|
| - cyclohexanone-oxime | 21.3% by weight |
| - residual cyclohexanone | less than 100 ppm. |

The hydrogen peroxide/cyclohexanone total molar ratio was equal to 1.08:1:
The cyclohexanone conversion was higher than 99.9%.
The cyclohexanone selectivity to cyclohexanone-oxime was 99.4%.
The hydrogen peroxide conversion was quantitative.
The hydrogen peroxide selectivity to oxime was 91.7%.

### EXAMPLE 5

Under the operative conditions of example 1, a 2 liter reactor was fed with:

| | |
|---|---|
| - cyclohexanone | 133.75 g/h |
| - t-butyl alcohol (12% H₂O) | 491.2 g/h |
| - hydrogen peroxide (50% by weight) (hydrogen peroxide/ketone feed molar ratio = 0.97:1) | 90 g/h |
| - gaseous ammonia: amount sufficient to maintain constant its concentration (about 2% by weight on the liquid medium). | |

The effluent, equal to 752 g/h, having the following composition:

| | |
|---|---|
| - cyclohexanone-oxime | 19.4% by weight |
| - cyclohexanone | 0.9% by weight |
| - water | 20.1% by weight |
| - ammonia | 2.0% by weight |

was passed to an exhaustion reactor of 1 liter volume, operating under the following conditions:

| | |
|---|---|
| - temperature | 85°C |
| - pressure | 1.8 bar |
| - residence time | 32 minutes |
| - suspended catalyst (calculated on the reaction medium) | 2% by weight. |

To the exhaustion reactor there were also fed 44 g/h of hydrogen peroxide at 10% by weight (hydrogen peroxide/ketone molar ratio = 1.87:1). The product leaving the reactor had the following composition:

| | |
|---|---|
| - cyclohexanone-oxime | 19.3% by weight |
| - cyclohexanone | 200 ppm |
| - water | 24.0% by weight |
| - ammonia | 1.5% by weight. |

The total hydrogen peroxide/cyclohexanone molar ratio was 1.6:1.
The cyclohexanone conversion was higher than 99.9%.
The ketone selectivity to oxime was equal to 99.3%.
The hydrogen peroxide conversion was quantitative.
The hydrogen peroxide selectivity to oxime was equal to 93%.

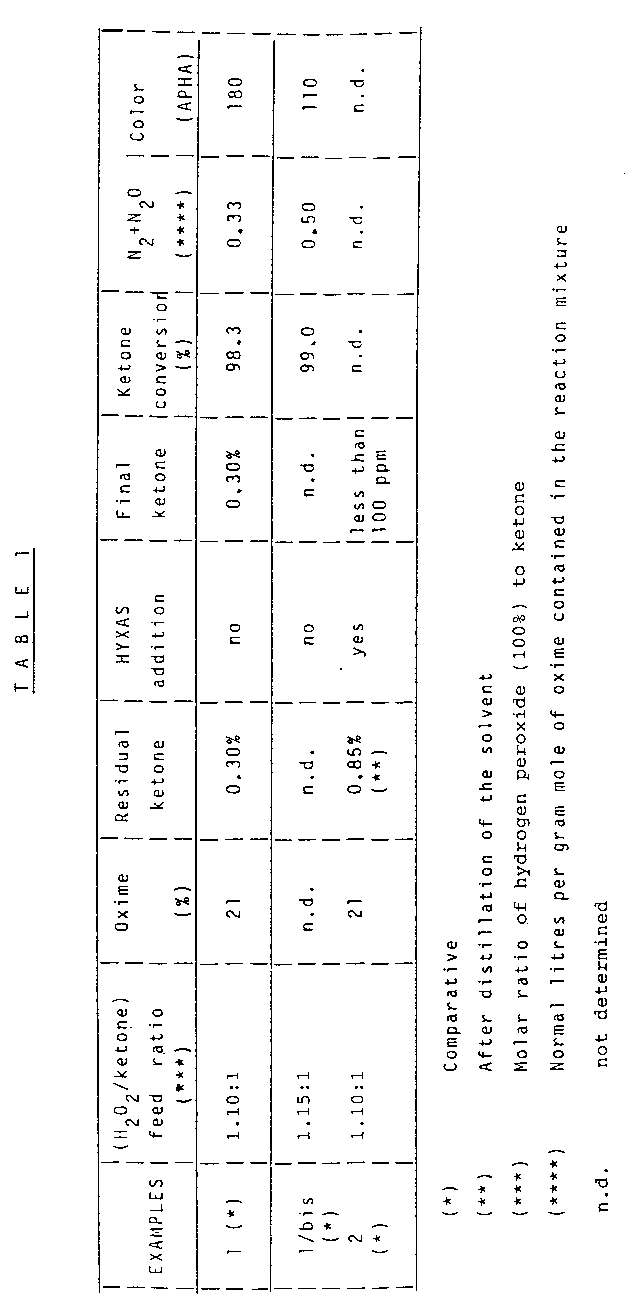

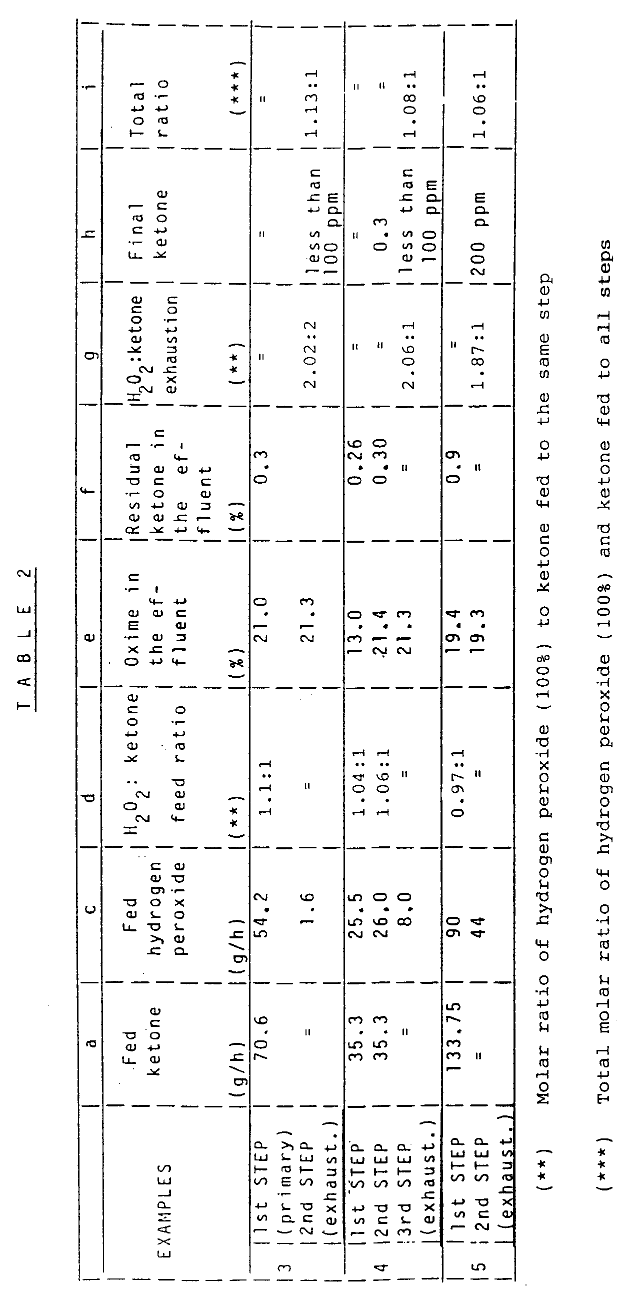

## Claims

1. A multistep process for the liquid phase ammoximation of carbonyl compounds with H₂O₂ and NH₃ at 60 to 100°C and 1.5 to 5 bar and in the presence of a catalyst based on silicon, titanium and oxygen, wherein
a) in one or more initial steps the molar ratio H₂O₂: carbonyl compound ranges from 0.9:1 to 1.15:1 (preferably from 1.0:1 to 1.1:1) and the carbonyl compound conversion is effected up to at least 95%, preferably up to 96 to 99%;
b) in a final step (exhaustion step) the molar ratio H₂O₂ : carbonyl compound ranges from 1.5:1 to 3.0:1, preferably from 1.5:1 to 2.2:1.

2. The process of claim 1, wherein
- the number of initial steps is 1 or 2; and/or
- the catalyst is titanium silicalite; and/or
- the residual carbonyl compound concentration in the effluent from the initial step(s) is equal to or lower than 1% (preferably ≦ 0.5%) by weight; and/or
- the ammonia concentration in the liquid reaction medium of all steps ranges from 1.0 to 2.5, preferably from 1.5 to 2.0% by weight; and/or
- the specific productivity in the initial step(s) ranges from 6 to 12 parts by weight of oxime per part of catalyst and per hour.

3. The process of any one of claims 1 and 2, wherein the temperature ranges form 70 to 90°C, the pressure ranges form 1.8 to 4 bar and the catalyst concentration ranges from 1 to 15% by weight, preferably from 1 to 6% by weight.

4. The process of any one of claims 1 to 3, wherein the specific productivity in the last step (exhaustion step) ranges from 0.1 to 5, preferably from 0.3 to 0.6 parts by weight of oxime per part of catalyst and per hour.

5. The process of any one of claims 1 to 4, conducted in the presence of an organic solvent preferably selected from t-butanol and toluene, the weight ratio of said solvent to said carbonyl compound ranging from 2.5:1 to 10:1.

6. The process of any one of claims 1 to 5, wherein the maximum oxime concentration in the liquid reaction medium ranges form 10 to 30% by weight, preferably from 20 to 25% by weight.

7. The process of any one of claims 1 to 6, wherein the catalyst particles suspended in the reaction liquid have an average size of from 1 to 100 »m, preferably of from 5 to 50 »m.

8. The process of any one of claims 1 to 7, wherein the carbonyl compound is selected from cyclohexanone, acetone, methyl ethyl ketone, acetophenone, cyclododecanone and enantic aldehyde.

9. The process of any one of claims 1 to 8, wherein the oxime is recovered from the reaction liquid, after the exhaustion step, by means of azeotropic distillation, followed by extraction with organic solvents, preferably toluene.

10. The process of any one of claims 1 to 9, wherein the reactor for each step is of the CSTR type and is equipped with a porous filtering element, the pores of which have an average size lower than the average size of the catalyst particles.

## Patentansprüche

1. Mehrstufenverfahren zur Ammoximation von Carbonylverbindungen in der flüssigen Phase mit H₂O₂ und NH₃ bei 60 bis 100 °C und 1.5 bis 5 bar und in Gegenwart eines Katalysators auf der Basis von Silicium, Titan und Sauerstoff, worin
a) in einem oder mehreren Anfangsschritten das Molverhältnis H₂O₂ : Carbonylverbindung im Bereich von 0.9 : 1 bis 1.15 : 1 (vorzugsweise 1.0 : 1 bis 1.1 : 1) liegt und die Umwandlung der Carbonylverbindung bis zu mindestens 95 %, vorzugsweise 96 bis 99 %, erfolgt,
b) in einem Endschritt (Erschöpfungsschritt) das Molverhältnis H₂O₂ : Carbonylverbindung im Bereich von 1.5 : 1 bis 3.0 : 1, vorzugsweise 1.5 : 1 bis 2.2 : 1, liegt.

2. Verfahren nach Anspruch 1, worin
- die Anzahl der Anfangsschritte 1 oder 2 ist und/oder
- der Katalysator Titansilikalit ist und/oder
- die Restkonzentration der Carbonylverbindung im Ausstrom aus dem (den) Angangsschritt(en) gleich oder niedriger als 1 Gew.-% (vorzugsweise ≦ 0.5 Gew.-%) ist und/oder die Ammoniakkonzentration im flüssigen Reaktionsmedium aller Schritte im Bereich von 1.0 bis 2.5, vorzugsweise 1.5 bis 2.0, Gew.-% liegt und/oder
- die spezifische Produktivität in dem (den) Anfangsschritt(en) im Bereich von 6 bis 12 Gew.-Teilen Oxim pro Teil Katalysator und pro Stunde liegt.

3. Verfahren gemäß irgendeinem der Ansprüche 1 und 2, worin die Temperatur im Bereich von 70 bis 90 °C, der Druck im Bereich von 1.8 bis 4 bar und die Katalysatorkonzentration im Bereich von 1 bis 15 Gew.-%, vorzugsweise 1 bis 6 Gew.-%, liegt.

4. Verfahren gemäß irgendeinem der Ansprüche 1 bis 3, worin die spezifische Produktivität im letzten Schritt (Erschöpfungsschritt) im Bereich von 0.1 bis 5, vorzugsweise 0.3 bis 0.6, Gew.-Teile Oxim pro Teil Katalysator und pro Stunde liegt.

5. Verfahren gemäß irgendeinem der Ansprüche 1 bis 4, durchgeführt in Gegenwart eines organischen Lösemittels, das vorzugsweise ausgewählt ist aus t-Butanol und Toluol, wobei das Gewichtsverhältnis des Lösemittels zur Carbonylverbindung im Bereich von 2.5 : 1 bis 10 : 1 liegt.

6. Verfahren gemäß irgendeinem der Ansprüche 1 bis 5, worin die maximale Oximkonzentration im flüssigen Reaktionsmedium im Bereich von 10 bis 30 Gew.-%, vorzugsweise 20 bis 25 Gew.-%, liegt.

7. Verfahren gemäß irgendeinem der Ansprüche 1 bis 6, worin die in der Reaktionsflüssigkeit suspendierten Katalysatorpartikel eine durchschnittliche Größe von 1 bis 100 »m, vorzugsweise 5 bis 50 »m, besitzen.

8. Verfahren gemäß irgendeinem der Ansprüche 1 bis 7, worin die Carbonylverbindung ausgewählt ist aus Cyclohexanon, Aceton, Methylethylketon, Acetophenon, Cyclododecanon und Önanthaldehyd.

9. Verfahren gemäß irgendeinem der Ansprüche 1 bis 8, worin das Oxim nach dem Erschöpfungsschritt durch azeotrope Destillation, gefolgt von einer Extraktion mit organischen Lösemitteln, vorzugsweise Toluol, aus der Reaktionsflüssigkeit gewonnen wird.

10. Verfahren gemäß irgendeinem der Ansprüche 1 bis 9, worin der Reaktor für jeden Schritt vom CSTR-Typ und ausgerüstet ist mit einem porösen Filtrierelement, dessen Poren eine durchschnittlich Größe besitzen, die geringer ist als die durchschnittliche Größe der Katalysatorpartikel.

## Revendications

1. Procédé à étapes multiples pour l'oximation en phase liquide de composés carbonylés avec du H₂O₂ et NH₃ à une température comprise entre 60 °C et 100 °C et sous une pression de 1,5 à 5 bar et en présence d'un catalyseur à base de silicium, de titane et d'oxygène, dans lequel
a) dans une ou plusieurs étapes initiales, le rapport molaire H₂O₂/composé carbonylé est compris entre 0,9:1 et 1,15:1 (de préférence entre 1,0:1 et 1,1:1) et la conversion du composé carbonylé est effectuée jusqu'à au moins 95 %, de préférence jusqu'à 96 - 99 %,
b) dans une étape finale (étape de parachèvement), le rapport molaire H₂O₂/composé carbonylé est compris entre 1,5:1 et 3,0:1, de préférence entre 1,5:1 et 2,2:1.

2. Procédé conforme à la revendication 1, dans lequel
- le nombre d'étapes initiales est égal à 1 ou 2, et/ou
- le catalyseur est la silicalite de titane, et/ou
- la concentration du composé carbonylé résiduel dans l'effluent de la ou des étape(s) initiale(s) est égale ou inférieure à 1 % (de préférence ≦ 0,5 %) en poids, et/ou
- la concentration d'ammonium dans le milieu réactionnel liquide de toutes les étapes est comprise entre 1,0 et 2,5, de préférence entre 1,5 et 2,0 % en poids, et/ou
- la productivité spécifique dans la ou les étape(s) initiale(s) est comprise entre 6 et 12 parties en poids d'oxime par partie de catalyseur et par heure.

3. Procédé conforme à une quelconque des revendications 1 et 2, dans lequel la température est comprise entre 70 et 90 °C, la pression est comprise entre 1,8 et 4 bar et la concentration du catalyseur est comprise entre 1 et 15 % en poids, de préférence entre 1 et 6 % en poids.

4. Procédé conforme à une quelconque des revendications 1 à 3 dans lequel la productivité spécifique dans la dernière étape (étape de parachèvement) est comprise entre 0,1 et 5, de préférence entre 0,3 et 0,6 parties en poids d'oxime par partie de catalyseur et par heure.

5. Procédé conforme à une quelconque des revendications 1 à 4, réalisé en présence d'un solvant organique choisi de préférence parmi le *t*-butanol et le toluène, le rapport en poids dudit solvant audit composé carbonylé étant compris entre 2,5:1 et 10:1.

6. Procédé conforme à une quelconque des revendications 1 à 5 dans lequel la concentration maximale en oxime dans le milieu réactionnel liquide est comprise entre 10 et 30 % en poids, de préférence entre 20 et 25 % en poids.

7. Procédé conforme à une quelconque des revendications 1 à 6 dans lequel les particules de catalyseur en suspension dans le milieu réactionnel liquide ont une taille moyenne comprise entre 1 et 100 »m, de préférence entre 5 et 50 »m.

8. Procédé conforme à une quelconque des revendications 1 à 7 dans lequel le composé carbonylé est choisi parmi la cyclohexanone, l'acétone, la méthyléthylcétone, l'acétophénone, la cyclododécanone et le *n*-heptanal.

9. Procédé conforme à une quelconque des revendications 1 à 8 dans lequel l'oxime est récupérée à partir du liquide réactionnel après l'étape de parachèvement, au moyen d'une distillation azéotropique suivie de l'extraction avec des solvants organiques, de préférence le toluène.

10. Procédé conforme à une quelconque des revendications 1 à 9 dans lequel le réacteur pour chaque étape est de type CSTR (*Continous Stirred Tank Reactor*, réacteur à agitation permanente) et est équipé d'un élément poreux de filtrage, dont les pores ont une taille moyenne inférieure à la taille moyenne des particules de catalyseur.
